# FASCICULE DE BREVET EUROPEEN

(11) **EP 2 201 385 B1**
(45) Date de publication et mention de la délivrance du brevet: **05.06.2013**
(21) Numéro de dépôt: 08862335.0
(22) Date de dépôt: 02.10.2008
(51) Int. Cl.: G01N 33/86, G01N 33/96

(54) **METHODE D'AJUSTEMENT DE LA CALIBRATION DE TESTS DIAGNOSTIQUES**
VERFAHREN ZUR EINSTELLUNG DER KALIBRIERUNG VON DIAGNOSETESTS
METHOD FOR ADJUSTING THE CALIBRATION OF DIAGNOSIS TESTS

(30) Priorité: 04.10.2007 FR 0706975
(43) Date de publication de la demande: 30.06.2010
(73) Titulaire: Diagnostica Stago, 92600 Asnières (FR)
(72) Inventeur: ESTEVE, Frédéric, F-75003 Paris (FR); NICOUD, Lydie, F-78170 La Celle Saint -Cloud (FR)
(74) Mandataire: Desaix, Anne
(86) Numéro de dépôt international: PCT/FR2008/001379
(87) Numéro de publication internationale: WO 2009/077678

(56) Documents cités:
- WO-A-95/12127
- WO-A-02/052276
- US-A- 5 138 034
- US-A1- 2005 136 449
- US-A1- 2007 020 765
- BRIEN W F ET AL: "In-house calibration of the International Sensitivity Index or calibration curve for determination of the international normalized ratio" ARCHIVES OF PATHOLOGY & LABORATORY MEDICINE, CHICAGO,IL, US, vol. 128, no. 3, 1 mars 2004 (2004-03-01), pages 308-312, XP002389073

## Description

La présente invention concerne une méthode directe ou indirecte d'ajustement de la calibration préétablie sur des réactifs destinés à réaliser des tests diagnostiques dans le domaine de la biologie médicale et plus particulièrement celui de l'hémostase.
L'hémostase est considérée comme un ensemble de mécanismes physiologiques qui concourent à la prévention et à l'arrêt des saignements. L'hémostase est souvent comparée à une balance, car la fluidité du sang est maintenue grâce à un équilibre entre activateurs et inhibiteurs du processus de coagulation.
Toute rupture de cet équilibre fera pencher la balance vers un processus pathologique : la thrombose, résultant de la formation d'un caillot pouvant provenir d'un déficit en inhibiteurs, ou l'hémorragie résultant de saignements pouvant être dus à un déficit en facteurs de la coagulation.
L'analyse et le dosage des facteurs activateurs ou inhibiteurs de la coagulation permet ainsi d'établir des diagnostics de prédisposition ou de risques de thrombose ou d'hémorragie dans différentes situations cliniques.
Ces tests sont réalisés pour la plupart en routine dans des laboratoires d'analyse ou des hôpitaux, de manière semi-automatisée ou automatisée. Généralement, selon les tests effectués et les instruments utilisés, les résultats consistent soit en un signal mesuré par l'instrument, soit en une activité biologique calculée par l'instrument.
Comme pour la plupart des tests biologiques réalisés sur des appareils d'analyse, il est nécessaire lorsque l'on effectue un test automatisé en hémostase, de disposer d'une calibration qui permette de calculer directement le taux ou l'activité du facteur étudié. Ces données de calibration sont obtenues généralement à l'aide de calibrants dont l'activité ou la concentration de l'analyte ciblé est bien caractérisée, et à partir desquels peuvent être établies des courbes de calibration permettant d'attribuer une concentration ou une activité à une intensité de signal donnée.

Or, dans la mesure où un même test peut souvent être réalisé sur des automates de gammes différentes, il est fréquent d'observer une variabilité inter-instruments. Cette variabilité ne permet pas toujours de comparer directement, pour un même test, des résultats obtenus sur des appareils différents qui utiliseraient toujours une même calibration standard.

Par ailleurs, comme beaucoup de réactifs se présentent sous forme liquide, ceux-ci peuvent être plus sensibles au phénomène de vieillissement que les réactifs sous forme lyophilisée. De ce fait, même si ces réactifs restent fonctionnels pendant un certain temps, ils peuvent néanmoins voir leur sensibilité diminuer en partie ou évoluer en fonction du temps.
Il en résulte alors une difficulté pour interpréter les résultats obtenus à partir d'un même réactif mais découlant de tests réalisés sur des périodes différentes.

La demande US 2007/0020765 décrit une méthode pour standardiser le temps de coagulation d'un échantillon, dans laquelle on utilise au moins deux calibrants pour lesquels des temps de coagulation standards ont été prédéterminés dans le même système de test que celui appliqué à l'échantillon, et à partir desquels on établit une courbe de calibration entre les temps standards prédéterminés et les temps réels mesurés pour ces mêmes calibrants, dans le test utilisé.
Le temps de coagulation mesuré dans l'échantillon est converti en temps de coagulation standardisé à l'aide de la courbe de calibration ainsi établie.
Cette méthode consiste donc à créer localement une calibration chaque fois que l'on utilise un nouveau kit pour mesurer un temps de coagulation dans un test d'hémostase.

La présente invention vise à fournir une méthode d'ajustement pour des instruments d'analyse et les réactifs précalibrés associés, ladite methode permettant de limiter ou d'éviter des fluctuations des résultats obtenus au cours du temps et/ou selon le type d'instrument utilisé pour les tests.

La présente invention a ainsi pour objet une méthode d'ajustement, soit du signal mesuré ou de l'activité calculée par l'instrument, soit d'une courbe de calibration préétablie (courbe de précalibration) pour un test d'exploration de la coagulation sanguine , réalisé sur un instrument avec des réactifs précalibrés, comprenant les étapes suivantes :
(i) établir une valeur de signal ou d'activité, dite valeur standard, pour un unique ou pour deux ajusteur(s) de calibration ayant une concentration ou une activité prédéterminée en un composé testé, et mesurer pour cet ou ces ajusteurs(s) l'écart entre la (ou les) valeur(s) standard de signal ou d'activité et la (ou les) valeur(s) soit du signal mesurée par l'instrument soit de la (ou des) valeur(s) d'activité calculée(s) à partir de la courbe de précalibration et, si l'écart mesuré a une valeur acceptable,
(ii) ajuster le signal mesuré par l'instrument ou la valeur de l'activité calculée par l'instrument ou ajuster (les) valeur(s) de la courbe de précalibration de manière à ce que la valeur du signal mesuré ou de l'activité calculée sur la courbe de précalibration pour l'unique ou les deux ajusteur(s) soit ramenée à une valeur de signal ou d'activité identique à la valeur de signal ou d'activité standard déterminée dans l'étape (i) pour l'ajusteur unique ou pour chacun des deux ajusteurs,
(iii) appliquer le même ajustement que celui décrit en (ii) à tous les échantillons testés dont le signal ou l'activité est mesuré avec lesdits réactifs précalibrés.

On entend par écart mesuré ayant une "valeur acceptable", un écart qui reste compris entre deux valeurs de la gamme des valeurs considérées comme admissibles par l'homme du métier, pour le réactif et l'instrument utilisés.

Cette différence peut être plus ou moins importante en fonction de la sensibilité du test mis en oeuvre et de l'ordre de grandeur des signaux mesurés.
De ce fait, la limite au-delà de laquelle l'ajustement de la précalibration ne sera plus acceptable doit être définie par rapport au type de test et d'instrument mis en oeuvre.
L'homme du métier qui est habitué à analyser les résultats fournis par les automates d'analyse peut facilement détecter lorsqu'il réalise des tests, les valeurs à partir desquelles les résultats sont considérés comme anormaux, que ce soit du fait du réactif utilisé ou de l'instrument.
Il est ainsi capable de définir des écarts entre les valeurs de signal ou d'activité standard et les valeurs de signal mesuré par l'instrument ou d'activité calculée sur la courbe de précalibration selon l'étape (i) de la méthode de l'invention, qui soient suffisamment faibles pour ne pas masquer des anomalies des réactifs ou des appareils utilisés.
De préférence, l'écart mesuré est d'au maximum 20 à 30 %.

Selon une variante préférée, la méthode de l'invention est réalisée avec un seul ajusteur.

Selon un mode de réalisation particulier de l'invention, l'ajustement, au moyen d'un unique ajusteur, d'un signal mesuré dans le cadre d'un test déterminé, conformément aux étapes (i) et (ii) du procédé selon l'invention peut être réalisé comme suit :
- pour l'étape (i) d'établissement de la valeur standard on opère de la façon suivante :

La valeur de signal standard assignée à l'ajusteur est calculée à partir des résultats établis au moment de l'étape de détermination de la précalibration du produit considéré. La détermination de la précalibration consiste à passer des étalons à taux connus de l'analyte à doser (composé testé) sur un panel dé plusieurs instruments. Chacun des étalons est passé en double, en triple ou en quadruple sur chaque instrument.

Dans le même temps, l'ajusteur à titrer en signal est passé lui aussi simultanément en double, en triple ou en quadruple sur ces mêmes instruments.

La moyenne arithmétique, la moyenne tronquée ou la médiane sont ensuite calculées en dehors de l'instrument sur toutes les valeurs de signal ainsi obtenues, séparément pour chaque étalon et pour l'ajusteur.

La détermination de l'équation de précalibration est calculée en dehors de l'instrument par régression des moindres carrés sur les différents couples (signal moyen ou médian ; concentration de l'étalon).

La valeur de signal standard de l'ajusteur correspond respectivement à la moyenne arithmétique, à la moyenne tronquée ou à la médiane de toutes les valeurs de signal mesurées sur cet ajusteur dans le cadre de cette détermination de la précalibration selon le mode de calcul retenu. La valeur de signal standard ainsi calculée pour cet ajusteur n'est valable que pour le lot particulier de produit précalibré.

La détermination de la valeur standard de signal ou de l'activité (cf plus loin) de l'ajusteur ou des ajusteurs peut donc être faite préalablement et indépendamment du test de diagnostic. La valeur standard de signal (ou de l'activité) de l'ajusteur (ou des ajusteurs) peut donc être fournie à l'utilisateur du test diagnostique avec le lot de réactifs à utiliser et l'ajusteur (les ajusteurs).
- pour les étapes (ii) et (iii) d'ajustement des valeurs mesurées de l'ajusteur et des échantillons testés, on procède comme suit :

La valeur de signal standard assignée à l'ajusteur pour son lot de produit précalibré est notée S. Lorsque cet ajusteur est passé en double, en triple ou en quadruple sur un instrument avec ce lot de produit précalibré, l'instrument calcule le signal T qui correspond, selon le mode de calcul choisi pour déterminer S, à la moyenne arithmétique, à la moyenne tronquée ou à la médiane de ces différents signaux mesurés. Si l'écart entre S et T est conforme aux critères d'acceptation connus par l'instrument, l'instrument calcule un coefficient d'ajustement C qui est égal au ratio de S sur T (C= S / T). Ainsi, en multipliant le signal T mesuré par l'instrument pour l'ajusteur par ce coefficient d'ajustement C égal à S / T, la valeur ajustée du signal de l'ajusteur est égale à S. De la même manière, lorsque les échantillons à doser sont passés sur ce même instrument, les mesures de signal obtenues sont ajustées en multipliant ces mesures par ce coefficient d'ajustement C. Ensuite, le calcul de la concentration ou de l'activité en analyte à doser est mis en oeuvre en appliquant la précalibration à cette valeur ajustée du signal.

Selon un autre mode de réalisation particulier de l'invention, l'ajustement au moyen d'un unique ajusteur est réalisé sur l'activité calculée par l'instrument d'analyse, conformément aux étapes (i) et (ii) du procédé selon l'invention, de la façon suivante :
- pour l'étape (i) d'établissement de la valeur standard

La valeur d'activité standard est assignée à l'ajusteur par une titration. On réalise pour ce faire plusieurs tests avec un ou différents lots de réactifs précalibrés sur différents automates. Le nombre de déterminations peut varier suivant le paramètre considéré mais est toujours supérieur ou égal à 3. Cette titration est distincte de la détermination de la précalibration.

L'ajusteur (qui est par exemple un réactif lyophilisé à base de plasma excipienté de type contrôle/calibrant)) est testé en double, en triple ou en quadruple lors de chaque détermination. Son activité standard est déduite par lecture des signaux mesurés sur la précalibration.

La moyenne arithmétique, la moyenne tronquée ou la médiane est ensuite calculée en dehors des instruments sur toutes les activités obtenues avec chaque lot de réactif précalibré sur chaque automate pour chaque ajusteur. La valeur d'activité standard ou titre de l'ajusteur correspond, selon le mode de calcul adopté, à la moyenne arithmétique, à la moyenne tronquée ou à la médiane de toutes les valeurs d'activités mesurées sur cet ajusteur dans le cadre de cette titration. La valeur d'activité standard ainsi calculée pour cet ajusteur peut être valable pour un ou plusieurs lots de produit précalibré.

Chaque fois que cela est possible, la titration de l'ajusteur est corrélée au standard international du paramètre. Cela permet de garantir, en plus de l'homogénéité dans le système considéré, la cohérence avec le système international.
- pour les étapes (ii) et (iii) d'ajustement de l'activité mesurée :

Le titre assigné à l'ajusteur est noté S. Lorsque cet ajusteur est passé en double, en triple ou en quadruple sur un instrument avec le ou les lots de produits précalibrés qui lui correspondent, l'instrument calcule, à partir du signal mesuré, l'activité T qui correspond selon le mode de calcul retenu pour déterminer S, à la moyenne arithmétique, à la moyenne tronquée ou à la médiane de ces différents signaux mesurés. Si l'écart entre S et T est conforme aux critères d'acceptation connus par l'instrument, l'instrument calcule un coefficient d'ajustement C qui est égal au ratio de S sur T (C= S / T). Ainsi, en multipliant le signal T mesuré par l'instrument pour l'ajusteur par ce coefficient d'ajustement C égal à S / T, la valeur ajustée de l'activité de l'ajusteur est égale à S. De la même manière, lorsque les échantillons à doser sont passés sur ce même instrument, les signaux mesurés sont convertis en activités grâce à l'équation de précalibration du réactif. Ces activités obtenues sont ensuite ajustées en les multipliant par ce coefficient d'ajustement C.

Un ajusteur utilisé dans le cadre de la méthode de l'invention est une composition qui permet :
- lors de l'utilisation d'un nouveau lot de réactifs, d'ajuster les résultats d'analyse rendus, à l'instrument mis en oeuvre pour réaliser le test tout en utilisant la précalibration. L'objectif est de recentrer les contrôles lorsqu'ils sont systématiquement décalés.
- au fur et à mesure du temps, de compenser une perte de sensibilité du réactif par exemple due à son vieillissement. Comme cela a été indiqué plus haut, ce point concerne particulièrement les réactifs liquides qui sont moins stables au cours du temps que les réactifs lyophilisés.
La composition de l'ajusteur en facteurs de la coagulation ou autres composants est définie en fonction du test réalisé.
L'ajusteur doit contenir au moins le composé étudié dans le test mis en oeuvre, dont la concentration ou l'activité est prédéterminée.
Dans le cas d'un test d'exploration de la coagulation sanguine, l'ajusteur est de préférence constitué par du plasma lyophilisé stabilisé ou par un pool de plasmas lyophilisés stabilisés.
Il peut également être constitué par des protéines plasmatiques purifiées ou semi-purifiées, lesdites protéines constituant l'apport des facteurs nécessaires au déroulement de la réaction produite dans le cadre du test réalisé et comprenant en outre le composé étudié (encore appelé facteur étudié), dont la concentration ou l'activité est prédéterminée.

La concentration (ou le taux d'activité) du facteur étudié, utilisée dans l'ajusteur doit être choisie de façon à réduire au maximum l'influence d'un éventuel bruit de fond sur la correction de la précalibration.
Pour ce faire, la concentration ou l'activité du composé testé de l'ajusteur est fixée de façon à ce que le rapport signal / bruit de fond soit élevé.
Selon le type de test mis en oeuvre, la relation entre le signal mesuré et l'activité ou la concentration du composé étudié de l'ajusteur est différente. De ce fait, on obtient des courbes dont les profils sont différents selon les tests.

Par exemple, dans le cas d'un dosage de l'antithrombine (AT) tel que celui proposé par le kit STA-Stachrom ATIII, commercialisé par Diagnostica Stago, la DDO (delta DO) diminue quand la concentration en ATIII augmente.
Or, la concentration en ATIII étant plus faible en situation pathologique qu'en situation normale, la concentration en AT de l'ajusteur pour la mise en oeuvre de la méthode d'ajustement selon l'invention, sera choisie pour que l'ajusteur soit positionné dans une zone couvrant une valeur pathologique (i.e. faible quantité d'ATIII).
Inversement, dans le cas d'un dosage de la protéine C tel que celui proposé par le kit STA-Stachrom Protein C (Diagnostica Stago), l'ajusteur sera positionné sur une zone correspondant à la normalité de la concentration en Protéine C, car le signal mesuré augmente proportionnellement avec la quantité de protéine C, et la zone de normalité est délimitée par les valeurs supérieures en taux de protéine C.
Dans le cas du dosage du D-Dimère comme par exemple celui réalisé par le kit STA-Liatest D-Di (Diagnostica Stago), on observe que le signal augmente avec la quantité de D-Di. Or, en situation de normalité, le taux de D-Dimère est plus faible qu'en situation pathologique. L'ajusteur selon l'invention sera donc choisi de façon à ce que le signal observé soit positionné en zone pathologique. Dans le cas du dosage au moyen du kit STA-Liatest, l'ajustement peut avoir la composition d'un réactif STA Liatest Control P ou d'un STA Liatest Control N.

La méthode selon l'invention peut être mise en oeuvre pour tout type de test d'exploration de la coagulation sanguine tel qu'un TCA (temps de Céphaline Activée), un TP (temps de Prothrombine ou temps de Quick) ou un TT (temps de Thrombine), ou tout autre test enzymologique classique basé sur des mesures de densité optique (DO) ou de temps.
Elle peut également être appliquée à des tests de type dosage immunologique en phase solide dispersée, basés sur la mesure photométrique de l'augmentation de turbidité d'une suspension de microsphères.
Les exemples ci-après illustrent la présente invention.

### Exemple n°1 :

### Etude de l'ajustement de la précalibration du STA Liatest D-Di : Faisabilité de la correction des DDO en un point sur la perte de réactivité liée au vieillissement du réactif.

La méthode de l'invention est appliquée sur un test commercialisé sous le nom STA-Liatest D-Di par la société Diagnostica Stago.
Il s'agit d'un kit de dosage des D-Dimères plasmatiques (D-Di) par immuno-turbidimétrie à l'aide de microsphères de latex recouvertes par deux anticorps monoclonaux anti-D-Dimères.
En présence de l'analyte (D-Dimère), la réaction de type antigène/anticorps entraîne une agglutination des particules de latex, qui induit une augmentation de la turbidité du mélange réactionnel.
Celle-ci est mesurée sur un analyseur de la gamme STA (Diagnostica Stago) en mode cinétique à 540 nm pendant 140 secondes, conformément aux indications de la notice du kit.
L'augmentation de l'absorbance est proportionnelle à la concentration en D-Dimères de l'échantillon testé.
Les réactifs du kit sont liquides, prêts à l'emploi.
Pour chaque lot de réactifs, une précalibration est fournie par papillon code-barré. Les coefficients de la précalibration du lot utilisé sont ainsi directement transmis à l'instrument par simple lecture du papillon code-barré.
Des plasmas de contrôle (contrôle Positif et contrôle Négatif) permettent la validation de l'étalonnage et des séries de dosage.
La mesure de la concentration en D-Di a été réalisée une première fois à T=0 (Tableau 1) sur 4 échantillons de plasma avec un lot spécifique de STA Liatest D-Di, puis une seconde fois sur les mêmes échantillons et avec le même lot, 20 mois plus tard (à T=20 mois) (Tableau 2).
Les plasmas utilisés sont des plasmas de niveau dont les valeurs cibles pour le paramètre mesuré (D-Di) sont déterminées : (valeur cible = valeur moyenne établie sur un grand nombre de tests expérimentaux)
- RIN2 est un plasma dont la valeur cible en D-Di est à 0,58 µg/ml
- PN2 est un plasma dont la valeur cible en D-Di est à 0,83 µg/ml.
- LCP est un plasma contrôle pathologique (STA Liatest Control P), ciblé à 2,40 µg/ml en D-Di.
- P2 est un autre plasma pathologique.
Le point d'ajustement utilisé dans cet exemple est le « STA Liatest Control P » (LCP).
Les DDO sont mesurées sur 10 intruments de la gamme STA.
Les DDO mesurées par chaque instrument ont été corrigées par un coefficient qui est calculé par le ratio de la DDO standard de ce réactif, égale à 0,129, sur la DDO moyenne d'un quadruplet (X4) obtenue sur chaque instrument.
Cette DDO standard de 0,129 correspond à la moyenne des DDO obtenues sur un lot de STA Liatest Control P (LCP dans cet exemple), lors de la détermination de la précalibration du lot de STA Liatest D-Di utilisé.
Quatre niveaux de concentration ont été testés sur un lot STA Liatest D-Di spécifique précalibré à T=0.
Les tableaux 1 et 2 donnent respectivement les résultats obtenus à T=0 (tableau 1) et à T=20 mois (tableau 2).
Les résultats du tableau 3, qui sont ceux obtenus à T=20 mois après ajustement par le LCP, sont très proches de ceux obtenus initialement à T=0 (tableau 1).

En conclusion : l'ajustement en un point des DDO permet de retrouver 20 mois plus tard avec le réactif précalibré à T=0 (Tableau 3) des concentrations moyennes équivalentes à celles obtenues à T=0 (Tableau 1).
L'ajustement en un point des DDO mesurées est donc efficace pour compenser la perte de sensibilité du STA Liatest D-Di.

**Tableau 1 : Concentrations en D-Dimère (µg/ml) obtenues à T=0 avec le réactif précalibré**

| | **RIN2** | **PN2** | **P2** | **LCP** |
|---|---|---|---|---|
| **MIN** | 0,55 | 0,79 | 2,30 | 2,33 |
| **MAX** | 0,66 | 0,88 | 2,56 | 2,53 |
| **MOY** | 0,59 | 0,84 | 2,46 | 2,45 |
| **SD** | 0,032 | 0,032 | 0,079 | 0,067 |
| **CV (%)** | 5,47 | 3,86 | 3,20 | 2,73 |

**Tableau 2 : Concentrations en D-Dimère (µg/ml) obtenues à T=20 mois avec le réactif précalibré**

| | **RIN2** | **PN2** | **P2** | **LCP** |
|---|---|---|---|---|
| **MIN** | 0,50 | 0,71 | 1,98 | 2,00 |
| **MAX** | 0,60 | 0,79 | 2,19 | 2,17 |
| **MOY** | 0,54 | 0,75 | 2,11 | 2,10 |
| **SD** | 0,029 | 0,028 | 0,064 | 0,054 |
| **CV (%)** | 5,43 | 3,77 | 3,04 | 2,59 |

**Tableau 3 : Concentrations en D-Dimère (µg/ml) obtenues à T=20 mois avec le réactif précalibré et après ajustement des DDO sur chaque instrument par le LCP**

| | **RIN2** | **PN2** | **P2** | **LCP** |
|---|---|---|---|---|
| **MIN** | 0,57 | 0,82 | 2,40 | **REF** |
| **MAX** | 0,66 | 0,90 | 2,61 | |
| **MOY** | 0,60 | 0,85 | 2,49 | |
| **SD** | 0,028 | 0,028 | 0,070 | |
| **CV (%)** | 4,67 | 3,29 | 2,82 | |

### Exemple n°2 :

### Etude de l'ajustement de la précalibration pour le STA Liatest Free PS.

L'étude est réalisée à partir d'un autre kit commercialisé par Diagnostica Stago, le STA Liatest Free PS.
Il s'agit d'un kit du même type que le précédent, qui permet un dosage quantitatif de la protéine S libre par méthode immuno-turbidimétrique.
La mesure est effectuée sur 10 instruments STA
Elle est réalisée sur différents lots de plasma, à savoir :
- Un plasma contrôle normal et un plasma contrôle pathologique (LCN et LCP : STA Liatest Control N+P -).
- Un plasma à 53 % : il s'agit d'un plasma pathologique lyophilisé.
- Un pool congelé de plasmas unitaires (pool Stago).
- Un plasma unitaire congelé.
Le tableau 4 indique les concentrations en protéine S libre (%) obtenues avec la calibration de chaque appareil par un calibrant interne.
Le tableau 5 indique les concentrations en protéine S libre (%) obtenues avec la précalibration commerciale appliquée sans ajustement : précalibration non ajustée.
Le tableau 6 indique les concentrations en protéine S libre (%) obtenues avec la précalibration commerciale après ajustement des DDO en un point sur le pool Stago : précalibration ajustée.

**Tableau 4 : Concentrations en Protéine S libre (%) obtenues avec la calibration de chaque instrument par un calibrant interne**

| **Résultats** | **LCP** | **Plasma 53 %** | **LCN** | **Pool Stago** | **Plasma unitaire** |
|---|---|---|---|---|---|
| **MIN** | 25.07 | 50.07 | 78.20 | 88.24 | 86.71 |
| **MAX** | 27.09 | 55.82 | 84.85 | 97.89 | 113.21 |
| **MOY** | 25.75 | 53.00 | 80.88 | 93.96 | 103.54 |
| **SD** | 0.63 | 1.80 | 2.32 | 2.81 | 7.63 |
| **CV** | 2.45 | 3.40 | 2.87 | 2.99 | 7.36 |

**Tableau 5 : Concentrations en Protéine S libre (%) obtenues avec la précalibration commerciale appliquée sans aucun ajustement**

| **Résultats** | **LCP** | **Plasma 53 %** | **LCN** | **Pool Stago** | **Plasma unitaire** |
|---|---|---|---|---|---|
| **MIN** | 24.35 | 46.77 | 70.57 | 81.89 | 77.84 |
| **MAX** | 27.51 | 57.79 | 94.48 | 104.71 | 125.35 |
| **MOY** | 26.05 | 52.72 | 80.58 | 93.79 | 103.82 |
| **SD** | 0.92 | 3.49 | 7.39 | 7.29 | 12.18 |
| **CV** | 3.55 | 6.62 | 9.16 | 7.77 | 11.74 |

**Tableau 6 : Concentrations en Protéine S libre obtenues avec la précalibration commerciale après ajustement des DDO et en un point sur le pool Stago**

| **Résultats** | **LCP** | **Plasma 53 %** | **LCN** | **Pool Stago** | **Plasma unitaire** |
|---|---|---|---|---|---|
| **MIN** | 25.11 | 51.03 | 77.05 | REF | 88.86 |
| **MAX** | 26.78 | 54.08 | 84.78 | | 113.03 |
| **MOY** | 26.07 | 52.68 | 80.24 | | 103.47 |
| **SD** | 0,58 | 1,04 | 2,45 | | 6,87 |
| **CV** | 2,22 | 1,97 | 3,06 | | 6,64 |

La variabilité inter-instruments mesurée sur 10 STA pour des concentrations supérieures à 50 % est très marquée avec le STA Liatest Free PS précalibré (précalibration non ajustée). Ainsi, le CV inter-instruments sur LCN est de 9.16 % sur la précalibration non ajustée contre 2.87 % sur la calibration de chaque instrument et 2,45 % sur la précalibration ajustée.
De la même manière, la variabilité inter-instruments est améliorée sur le plasma à 53 % (dans la zone décisionnelle) et sur le LCP à 26 % lorsque la précalibration est ajustée :
*Plasma à 53 % : CVcal. instrument = 3.40 % . CVprécal. non ajustée* = *6.62 %, CVprecal. ajustée = 1.97 %.*
*Plasma LCP à 26 % : CVcal. instrument = 2.45 % . CVprécal. non ajustée = 3, 45 %, CVprecaL ajustée = 2, 22 %.*

En conséquence, l'ajustement en un point de la précalibration permet de retrouver, à tous les niveaux de concentration, des CV inter-instruments :
- meilleurs que ceux obtenus avec la précalibration non ajustée
- au moins équivalents à ceux obtenus avec une calibration de chaque instrument par un calibrant interne.

En conclusion, la variabilité inter-instruments est nettement améliorée sur le STA Liatest Free PS par la méthode d'ajustement en un point des DDO selon l'invention.

### Exemple n°3 :

Etude de l'ajustement de précalibration du STA-Fib 2 : faisabilité de la correction des taux (g/l) sur le centrage des contrôles (valeur obtenue la plus proche de la valeur cible = centre de la fourchette fournie pour un système donné).

La méthode de l'invention a été appliquée sur un test commercialisé sous le nom STA-Fib 2 par la société Diagnostica Stago.
Il s'agit d'un kit de dosage du fibrinogène plasmatique selon la méthode de Clauss.

En présence d'un excès de thrombine, le temps de coagulation d'un plasma, dilué dans des proportions adéquates, est inversement proportionnel au taux de fibrinogène plasmatique.

Le réactif du kit est lyophilisé. Pour chaque lot de réactif, une précalibration est fournie par papillon code-barré. Les coefficients de la précalibration du lot utilisé sont ainsi directement transmis à l'instrument par simple lecture du papillon code-barré.

Des plasmas de contrôles (normal et hypofibrinogénémique) permettent la validation de l'étalonnage et des séries de dosage.

La mesure du taux de fibrinogène plasmatique a été réalisée sur différents plasmas de contrôles sur un parc d'automates de 71 machines dont une quarantaine de STA-R, une dizaine de STAc et une dizaine de STA-Satellite.

Les contrôles utilisés sont des plasmas de niveau dont les valeurs cibles pour le paramètre mesuré sont les suivantes :
- CCN est un plasma dont la valeur cible est à 2,95 g/l
- CCP est un plasmas dont la valeur cible est à 1,30 g/l
- HYPER est un plasma dont la valeur cible est à 5,27 g/l

Le plasma servant d'ajusteur était le plasma AJP dont la valeur cible est de 3,20 g/l.

Pour cette étude, les taux ont été mesurés en n=5 sur chaque instrument testé. A partir des valeurs obtenues pour le plasma AJP sur chaque automate, les taux ont été ajustés (selon le procédé décrit dans la présente demande, pour l'ajustement sur l'activité calculée).

Les tableaux ci-dessous présentent les taux moyens obtenus avec et sans ajustement (taux obtenus grâce à la précalibration du lot concerné) par rapport à la valeur cible des contrôles testés ainsi que l'écart-type global observé.

| **Sans ajustement** | **CCN cible : 2,95 g/l** | **CCP cible : 1,30 g/l** | **HYPER cible : 5,27 g/l** |
|---|---|---|---|
| Moyenne | 2,82 | 1,24 | 5,06 |
| SD | 0,11 | 0,04 | 0,19 |

| **Avec ajustement** | **CCN cible : 2,95 g/l** | **CCP cible : 1,30 g/l** | **HYPER cible : 5,27 g/l** |
|---|---|---|---|
| Moyenne | 2,91 | 1,28 | 5,23 |
| SD | 0,11 | 0,05 | 0,19 |

En conclusion, l'ajustement réalisé a permis de recentrer les taux obtenus sur les différents plasmas vers leur valeur cible tout en conservant un écart- type homogène sur le parc testé.

## Revendications

1. Méthode d'ajustement, soit du signal mesuré ou de l'activité calculée par l'instrument, soit d'une courbe de calibration préétablie (courbe de précalibration) pour un test d'exploration de la coagulation sanguine, réalisé sur un instrument avec des réactifs précalibrés, comprenant les étapes suivantes :
(i) établir une valeur de signal ou d'activité standard pour un unique ou pour deux ajusteur(s) de calibration ayant une concentration ou une activité prédéterminée en un composé testé, mesurer pour cet ou ces ajusteur(s) l'écart entre la (ou les) valeur(s) de signal ou d'activité standard et la (ou les) valeur(s) soit du signal mesurée par l'instrument soit de la (ou des) valeur(s) d'activité calculée(s) à partir de la courbe de précalibration, et, si l'écart mesuré a une valeur acceptable,
(ii) ajuster le signal mesuré par l'instrument ou la valeur de l'activité calculée par l'instrument ou ajuster les valeurs de la courbe de précalibration de manière à ce que la valeur du signal mesuré ou de l'activité calculée sur la courbe de précalibration pour l'unique ou les deux ajusteur(s) soit ramenée à une valeur de signal ou d'activité identique à la valeur de signal ou d'activité standard déterminée dans l'étape (i) pour l'ajusteur unique ou pour chacun des deux ajusteurs,
(iii) appliquer le même ajustement que celui décrit en (ii) à tous les échantillons dont le signal ou l'activité est mesuré avec lesdits réactifs précalibrés.

2. Méthode selon la revendication 1, dans laquelle la concentration ou l'activité du composé testé de l'ajusteur est fixée de façon telle que le rapport signal/bruit soit élevé dans le test considéré.

3. Méthode d'ajustement selon la revendication 1 **caractérisée en ce que** l'on utilise un seul ajusteur.

4. Méthode d'ajustement selon la revendication 1 **caractérisée en ce que** l'écart mesuré selon l'étape (i) est d'au maximum 20 à 30%.

5. Méthode d'ajustement selon l'une quelconque des revendications 1 à 4, **caractérisée en ce que** la composition de l'ajusteur en facteurs de la coagulation est définie en fonction dudit test d'exploration de la coagulation, ledit ajusteur contenant au moins le composé étudié dans le test mise en oeuvre, dont la concentration ou l'activité a été prédéterminée.

6. Méthode selon l'une quelconque des revendications 1 à 5, **caractérisée en ce que** l'ajusteur est constitué par :
- du plasma lyophilisé stabilisé ou d'un pool de plasmas lyophilisés stabilisés ou,
- des protéines plasmatiques purifiées ou semi-purifiées, lesdites protéines constituant l'apport des facteurs nécessaires au déroulement de la réaction produite dans le cadre du test réalisé et comprenant en outre le composé étudié, dont la concentration ou l'activité est prédéterminée.

7. Méthode selon l'une quelconque des revendications 1 à 6, **caractérisée en ce que** le test d'exploration de la coagulation sanguine est un test de type dosage immunologique en phase solide dispersée.

8. Méthode selon l'une quelconque des revendications 1 à 7, dans laquelle le test d'exploration fonctionnelle est un test de dosage des D-Dimères.

9. Méthode selon l'une quelconque des revendications 1 à 6, dans laquelle le test d'exploration de la coagulation est un TCA, un TP, un TT ou tout autre test enzymologique classique.

10. Méthode selon l'une quelconque des revendications 1 à 9, dans laquelle l'ajusteur n'est pas utilisé pour préparer la courbe de précalibration.

11. Utilisation de la méthode selon l'une quelconque des revendications 1 à 10, pour corriger :
- l'influence de l'évolution des réactifs, notamment des réactifs liquides, utilisés dans un automate donné pour la mise en oeuvre d'un test diagnostique en biologie médicale, notamment du fait de la durée de conservation desdits réactifs, ou
- l'influence de l'instrument utilisé lors d'un test diagnostique en biologie médicale.

12. Utilisation d'un ajusteur de calibration dans une méthode d'ajustement d'un signal mesuré ou d'une activité calculée ou d'une courbe de calibration préétablie selon l'une quelconque des revendications 1 à 10, dans un test d'exploration de la coagulation sanguine caractérisé ce que l'ajusteur est choisi parmi :
- un plasma lyophilisé stabilisé ou d'un pool de plasmas lyophilisés stabilisés, dont la concentration ou l'activité en un composé testé dans un test d'exploration de la coagulation est prédéterminée, ou
- des protéines plasmatiques purifiées ou semi-purifiées, lesdites protéines constituant l'apport des facteurs nécessaires au déroulement de la réaction produite dans le cadre du test réalisé et comprenant en outre le composé étudié, dont la concentration ou l'activité est prédéterminée et
- ledit ajusteur étant tel que la concentration ou l'activité en composé testé est choisie dans une zone correspondant à un rapport signal / bruit élevé pour ledit test d'exploration.

## Patentansprüche

1. Verfahren zum Einstellen entweder des durch das Analysegerät gemessenen Signals bzw. der durch das Analysegerät berechneten Aktivität oder einer vorab festgelegten Kalibrierkurve (Präkalibrierkurve) für einen Test zur Untersuchung der Blutgerinnung, der an einem Analysegerät mit präkalibrierten Reagenzien durchgeführt wird, das die folgenden Schritte umfasst:
(i) Festlegen eines Signal- oder Aktivitätsstandardwertes für einen einzigen oder für zwei Kalibratoren mit einer vorbestimmten Konzentration oder einer vorbestimmten Aktivität in einer getesteten Verbindung, Messen der Abweichung zwischen dem (bzw. den) Signal- bzw. Aktivitätsstandardwert(en) und dem (bzw. den) mit dem Gerät gemessenen Signalwert(en) oder dem (bzw. den) anhand der Präkalibrierkurve berechneten Aktivitätswert(en) für den bzw. die Kalibratoren, und Bewerten, ob die gemessene Abweichung einen akzeptablen Wert hat,
(ii) Einstellen des mit dem Gerät gemessenen Signals oder des mit dem Gerät berechneten Aktivitätswertes oder Einstellen der Werte der Präkalibrierkurve, so dass der Wert des gemessenen Signals oder der anhand der Präkalibrierkurve berechneten Aktivität für den einzigen oder die beiden Kalibratoren auf einen Signal- oder Aktivitätswert gebracht wird, der mit dem Signal- bzw. Aktivitätsstandardwert identisch ist, der in Schritt (i) für den einzigen Kalibrator oder für jeden der beiden Kalibratoren bestimmt wurde,
(iii) Durchführen der gleichen Einstellung wie in (ii) beschrieben bei allen Proben, bei denen das Signal oder die Aktivität mit den präkalibrierten Reagenzien gemessen wird.

2. Verfahren nach Anspruch 1,
bei dem die Konzentration oder die Aktivität der getesteten Verbindung des Kalibrators so festgelegt ist, dass das Signal-Rausch-Verhältnis in dem betreffenden Test hoch ist.

3. Verfahren zum Einstellen nach Anspruch 1,
**dadurch gekennzeichnet, dass** ein einziger Kalibrator verwendet wird.

4. Verfahren zum Einstellen nach Anspruch 1,
**dadurch gekennzeichnet, dass** die in Schritt (i) gemessene Abweichung maximal 20 bis 30 % beträgt.

5. Verfahren zum Einstellen nach einem der Ansprüche 1 bis 4,
**dadurch gekennzeichnet, dass** die Zusammensetzung des Kalibrators aus Gerinnungsfaktoren in Abhängigkeit des Tests zur Gerinnungsuntersuchung festgelegt ist, wobei der Kalibrator wenigstens die in dem verwendeten Test untersuchte Verbindung enthält, deren Konzentration oder Aktivität vorher bestimmt wurde.

6. Verfahren nach einem der Ansprüche 1 bis 5,
**dadurch gekennzeichnet, dass** der Kalibrator gebildet ist von:
- stabilisiertem lyophilisiertem Plasma oder einem stabilisierten lyophilisierten Plasmapool oder,
- gereinigten oder halbgereinigten Plasmaproteinen, wobei die Proteine die Zufuhr der Faktoren darstellen, die für den Ablauf der Reaktion notwendig sind, zu der es im Rahmen des durchgeführten Tests kommt, und ferner die untersuchte Verbindung umfassen, deren Konzentration oder Aktivität vorbestimmt ist.

7. Verfahren nach einem der Ansprüche 1 bis 6,
**dadurch gekennzeichnet, dass** der Test zur Untersuchung der Blutgerinnung ein Test vom Typ Immunoassay in dispergierter Festphase.

8. Verfahren nach einem der Ansprüche 1 bis 7,
bei dem der funktionelle Untersuchungstest ein D-Dimer-Assay-Test ist.

9. Verfahren nach einem der Ansprüche 1 bis 6,
bei dem der Test zur Gerinnungsuntersuchung ein PTT, ein TPZ, ein TZ oder jeder andere herkömmliche Enzymtest ist.

10. Verfahren nach einem der Ansprüche 1 bis 9,
bei dem der Kalibrator nicht zur Vorbereitung der Präkalibrierkurve verwendet wird.

11. Verwendung des Verfahrens nach einem der Ansprüche 1 bis 10, zur Korrektur:
- des Einflusses der Veränderung von Reagenzien, insbesondere flüssiger Reagenzien, die in einem gegebenen Analyseautomat zur Durchführung eines diagnostischen Tests in der Labormedizin verwendet werden, insbesondere aufgrund der Lagerungszeit der Reagenzien, oder
- des Einflusses des bei einem diagnostischen Test in der Labormedizin verwendeten Analysegeräts.

12. Verwendung eines Kalibrators bei einem Verfahren zum Einstellen eines gemessenen Signals oder einer berechneten Aktivität oder einer vorab festgelegten Kalibrierkurve nach einem der Ansprüche 1 bis 10 bei einem Test zur Untersuchung der Blutgerinnung,
**dadurch gekennzeichnet, dass** der Kalibrator gewählt ist aus:
- einem stabilisierten lyophilisierten Plasma oder einem stabilisierten lyophilisierten Plasmapool, dessen Konzentration oder Aktivität in einer getesteten Verbindung in einem Test zur Gerinnungsuntersuchung vorbestimmt ist, oder
- gereinigten oder halbgereinigten Plasmaproteinen, wobei die Proteine die Zufuhr der Faktoren darstellen, die für den Ablauf der Reaktion notwendig sind, zu der es im Rahmen des durchgeführten Tests kommt, und ferner die untersuchte Verbindung umfassen, deren Konzentration oder Aktivität vorbestimmt ist, und
- wobei der Kalibrator derart ist, dass die Konzentration oder die Aktivität in der getesteten Verbindung aus einem Bereich gewählt ist, der einem hohen Signal-Rausch-Verhältnis für den Untersuchungstest entspricht.

## Claims

1. Method for adjusting either the signal measured or activity calculated by the instrument or a predefined calibration curve (pre-calibration curve) for a medical biology investigation test, performed on an instrument with pre-calibrated reagents, comprising the following steps:
(i) defining a signal or activity value, referred to as the standard value, for a single or for two calibration adjuster(s) having a predetermined concentration or activity of a compound under test, measuring for said adjuster(s) the difference between the standard signal or activity value(s) and the value(s) either of the signal measured by the instrument or the activity value(s) calculated musing the pre-calibration curve and, if the difference measured has an acceptable value,
(ii) adjusting the signal measured by the instrument or the value of the activity calculated by the instrument or adjusting the value(s) of the pre-calibration curve such that the value of the signal measured or activity calculated on the pre-calibration curve for the single or both adjuster(s) is brought back to a signal or activity value identical to the standard signal or activity value determined in step (i) for the single adjuster or for each of the two adjusters,
(iii) applying the same adjustment as that described in (ii) to all the samples tested the signal or activity of which is measured with said pre-calibrated reagents.

2. Method according to claim 1, wherein the test compound concentration or activity of the adjuster is defined such that the signal/noise ratio is high in the test in question.

3. Adjustment method according to claim 1 **characterised in that** a single adjuster is used.

4. Adjustment method according to claim 1 **characterised in that** the difference measured according to step (i) is not more than 20 to 30%.

5. Adjustment method according to any of claims 1 to 4, **characterised in that** the composition of the adjuster with respect to clotting factors is defined according to said clotting investigation test, said adjuster containing at least the compound studied in the test used, the concentration or activity whereof is predetermined.

6. Method according to any of claims 1 to 5, **characterised in that** the adjuster consists of:
- stabilised freeze-dried plasma or a stabilised freeze-dried plasma pool or,
- purified or semi-purified plasma proteins, said proteins providing the supply of factors required to perform the reaction produced as part of the test performed and further comprising the compound under study, the concentration or activity whereof is predetermined.

7. Method according to any of claims 1 to 6, **characterised in that** the blood clotting investigation test is a dispersed solid phase immunological assay test.

8. Method according to any of claims 1 to 7, wherein the functional investigation test is a D-Dimer assay test.

9. Method according to any of claims 1 to 6, wherein the clotting investigation test is an APTT, PT, TT test or any other conventional enzymological test.

10. Method according to any of claims 1 to 9, wherein the adjuster is not used to prepare the pre-calibration curve.

11. Use of the method according to any of claims 1 to 10, for correcting:
- the influence of the changes impacting reagents, particularly liquid reagents, used in a given automatic apparatus for the implementation of a medical biology diagnostic test, particularly due to the shelf-life of said reagents, or
- the influence of the instrument used for a medical biology diagnostic test.

12. Use of a calibration adjuster in a method for adjusting either the signal measured or an activity calculated or a predefined calibration curve according to any one of claims 1 to 10, in a blood clotting investigation test, **characterised in that** the adjuster is selected from:
- a stabilised freeze-dried plasma or a stabilised freeze-dried plasma pool, the concentration or activity whereof of a compound under test in a clotting investigation test is predetermined, or
- purified or semi-purified plasma proteins, said proteins providing the supply of factors required to perform the reaction produced as part of the test performed and further comprising the compound under study, the concentration or activity whereof is predetermined, and
- said adjuster being such that concentration or activity of the compound under test is selected in a range corresponding to a high signal / noise ratio for said investigation test.
